(19) Europäisches Patentamt
European Patent Office
Office européen des brevets

(11) **EP 1 874 720 B1**

(12) **FASCICULE DE BREVET EUROPEEN**

(45) Date de publication et mention
de la délivrance du brevet:
**06.03.2013 Bulletin 2013/10**

(21) Numéro de dépôt: **06743726.9**

(22) Date de dépôt: **24.04.2006**

(51) Int Cl.:
*C07C 51/235* *(2006.01)*    *C07C 57/04* *(2006.01)*

(86) Numéro de dépôt international:
**PCT/FR2006/000907**

(87) Numéro de publication internationale:
**WO 2006/114506 (02.11.2006 Gazette 2006/44)**

(54) **PROCEDE DE PREPARATION D'ACIDE ACRYLIQUE A PARTIR DE GLYCEROL**

VERFAHREN ZUR HERSTELLUNG VON ACRYLSÄURE AUS GLYCEROL

METHOD FOR PRODUCING ACRYLIC ACID FROM GLYCEROL

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **25.04.2005 FR 0504111**
**10.01.2006 FR 0600183**
**10.06.2005 US 689423 P**

(43) Date de publication de la demande:
**09.01.2008 Bulletin 2008/02**

(73) Titulaire: **ARKEMA FRANCE**
**92700 Colombes (FR)**

(72) Inventeurs:
• **DUBOIS, Jean-Luc**
**F-69390 Millery (FR)**
• **DUQUENNE, Christophe**
**F-80250 Chaussoy-Epagny (FR)**
• **HÖLDERICH, Wolfgang**
**67227 Frankenthal (DE)**

(74) Mandataire: **Bonnel, Claudine**
**ARKEMA FRANCE**
**Département Propriété Industrielle**
**420, rue d'Estienne d'Orves**
**92705 Colombes Cedex (FR)**

(56) Documents cités:
**EP-A- 0 959 062**    **EP-A- 1 156 027**
**FR-A- 695 931**    **US-A- 2 558 520**
**US-A- 5 387 720**

• **DATABASE WPI Section Ch, Week 200560 Derwent Publications Ltd., London, GB; Class A41, AN 2005-585659 XP002357723 & JP 2005 213225 A (NIPPON SHOKUBAI CO LTD) 11 août 2005 (2005-08-11)**

**Description**

La présente invention concerne une nouvelle voie de synthèse de l'acide acrylique, et a plus particulièrement pour objet la préparation d'acide acrylique à partir du glycérol en présence d'oxygène moléculaire.

Le principal procédé de synthèse classique de l'acide acrylique utilise une réaction catalytique du propylène à l'aide d'un mélange contenant de l'oxygène. Cette réaction est conduite généralement en phase vapeur, et le plus souvent en deux étapes :

- La première étape réalise l'oxydation sensiblement quantitative du propylène en un mélange riche en acroléine, dans lequel l'acide acrylique est minoritaire,
- La deuxième étape réalise l'oxydation sélective de l'acroléine en acide acrylique.

Les conditions de réaction de ces deux étapes, réalisées dans deux réacteurs en série, sont différentes et nécessitent des catalyseurs adaptés à la réaction. Il n'est pas nécessaire d'isoler l'acroléine au cours de ce procédé en deux étapes. La réaction peut également être conduite dans un seul réacteur, mais, dans ce cas, il est nécessaire de séparer et recycler de grandes quantités d'acroléine à l'étape d'oxydation.

Il est connu depuis longtemps que l'acroléine peut être obtenue à partir de glycérol. Le glycérol (appelé aussi glycérine) est issu de la méthanolyse des huiles végétales en même temps que les esters méthyliques qui sont eux employés notamment comme carburants ou combustibles dans le gazole et le fioul domestique. C'est un produit naturel qui jouit d'une aura "verte", il est disponible en grande quantité et peut être stocké et transporté sans difficulté. De nombreuses études sont consacrées à la valorisation du glycérol selon son degré de pureté, et la déshydratation du glycérol en acroléine est une des voies envisagées.

La réaction mise en jeu pour obtenir l'acroléine à partir du glycérol est :

$$CH_2OH-CHOH-CH_2OH \Leftrightarrow CH_2=CH-CHO + 2H_2O$$

En règle générale la réaction d'hydratation est favorisée aux basses températures, et la déshydratation est favorisée aux températures élevées. Pour obtenir l'acroléine, il faut donc mettre en oeuvre une température suffisante, et/ou un vide partiel pour déplacer la réaction. La réaction peut être effectuée en phase liquide ou en phase gaz. Ce type de réaction est connu pour être catalysée par des acides. Différents procédés de synthèse de l'acroléine à partir du glycérol sont décrits dans la littérature : FR 695931; US 2,558,520 ; WO 99/05085 ; US 5,387,720.

La réaction de déshydratation du glycérol en acroléine s'accompagne généralement de réactions secondaires entraînant la formation de sous-produits tels que l'hydroxypropanone, le propanaldéhyde, l'acétaldéhyde, l'acétone, des produits d'addition de l'acroléine sur le glycérol, des produits de polycondensation du glycérol, des éthers de glycérol cycliques.

Dans la demande internationale WO 2005/073160, le produit de réaction résultant de la réaction de déshydratation du glycérol en phase gaz, est soumis à une étape ultérieure d'oxydation en phase gaz pour obtenir de l'acide acrylique. Ce procédé en deux étapes est mis en oeuvre avec du glycérol pur ou des solutions aqueuses concentrées comportant plus de 50% en poids de glycérol. Des rendements en acide acrylique se situant entre 55% et 65% sont obtenus en utilisant un catalyseur à base d'alumine imprégnée d'acide phosphorique et de silice pour la première étape de déshydratation, et un oxyde mixte Mo-V-W-Cu-O supporté sur de l'alumine pour la seconde étape d'oxydation. Le procédé est mis en oeuvre dans deux réacteurs en série, de l'oxygène pouvant être ajouté facilement au mélange gazeux alimentant le second réacteur. Le procédé peut être mis en oeuvre aussi dans un seul réacteur contenant, soit les deux catalyseurs empilés définissant deux zones de réaction, soit les deux catalyseurs en mélange, cette configuration présentant l'inconvénient de désactiver plus rapidement l'un des deux catalyseurs, ce qui rend difficile la maintenance du procédé.

De façon surprenante, la Société Déposante a découvert qu'il est possible d'obtenir de l'acide acrylique directement à partir de glycérol, lorsque l'on effectue la réaction de déshydratation du glycérol en présence d'oxygène et avec des solutions aqueuses diluées de glycérol. La présence d'oxygène permet de réaliser, en plus de la réaction de déshydratation, une réaction d'oxydation, offrant la possibilité d'obtenir en une seule étape la formation d'acide acrylique à partir du glycérol. L'apport d'oxygène dans le milieu réactionnel a aussi un effet bénéfique pour réduire la formation de coke sur le catalyseur mis en oeuvre et inhiber sa désactivation, et limiter la formation de sous-produits tels que le propanaldéhyde, l'acétone, le phénol et l'hydroxypropanone.

Un procédé de production d'acide acrylique directement à partir de glycérol est particulièrement avantageux puisqu'il permet de réaliser la synthèse en un seul réacteur et ainsi de réduire les investissements nécessaires.

Le principe du procédé selon l'invention est basé sur les 2 réactions consécutives de déshydratation et d'oxydation :

$$CH_2OH\text{-}CHOH\text{-}CH_2OH \leftrightarrow CH_2=CH\text{-}CHO + 2H_2O$$

$$CH_2=CH\text{-}CHO + \tfrac{1}{2} O_2 \rightarrow CH_2=CH\text{-}COOH$$

Dans ce schéma réactionnel, appelé oxydéshydratation, l'exothermicité de la réaction d'oxydation est combinée avec l'endothermicité de la réaction de déshydratation, ce qui contribue à un meilleur équilibre thermique du procédé.

Un autre avantage de ce procédé réside dans le fait qu'il n'est pas dépendant d'une matière première d'origine fossile, comme le propylène, mais qu'il utilise une matière première renouvelable ; un tel procédé répond donc aux critères associés au nouveau concept de "chimie verte", dans un cadre plus global de développement durable.

La présente invention a donc pour objet un procédé de fabrication d'acide acrylique en une étape par réaction d'oxydéshydratation du glycérol en présence d'oxygène moléculaire, le glycérol étant sous forme de solution aqueuse de concentration comprise entre 10% et 50% en poids dans le réacteur

L'oxygène moléculaire peut être présent sous forme d'air ou sous forme d'un mélange de gaz contenant de l'oxygène moléculaire. La quantité d'oxygène est choisie de façon à être en dehors du domaine d'inflammabilité en tout point de l'installation. La teneur en oxygène dans le procédé selon l'invention sera en général choisie de façon à ne pas dépasser 20% par rapport au mélange de gaz en entrée de réaction (mélange glycérol/$H_2O$/oxygène/gaz inertes).

La réaction de déshydratation du glycérol en acroléine est généralement effectuée sur des catalyseurs solides acides. Ces catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides. Les catalyseurs préférés sont les zircones sulfatées, les zircones phosphatées, les zircones tungstées, les zircones silicées, les oxydes de titane ou d'étain sulfatés, les alumines ou silices phosphatées. Ces catalyseurs acides, adaptés pour la réaction de déshydratation du glycérol, facilitent la désorption de l'acide acrylique produit, mais ne sont pas optimisés pour des réactions d'oxydation ; le rendement en acide acrylique est alors limité avec ce type de catalyseur.

Il est préférable donc, pour obtenir une bonne sélectivité en acide acrylique, que le solide mis en oeuvre soit capable aussi de catalyser la réaction d'oxydation. On choisira comme catalyseur d'oxydation, des solides contenant au moins un élément choisi dans la liste Mo, V, W, Re, Cr, Mn, Fe, Co, Ni, Cu, Zn, Sn, Te, Sb, Bi, Pt, Pd, Ru, Rh, présent sous la forme métallique ou sous forme d'oxyde, de sulfate ou de phosphate. Il est possible d'envisager d'utiliser un catalyseur unique capable de catalyser à la fois la réaction de déshydratation et la réaction d'oxydation, mais aussi de disposer d'un mélange de catalyseurs, chacun étant capable d'effectuer l'une des deux réactions de façon optimale. Ce mélange de catalyseurs est alors, soit un mélange intime de catalyseurs, soit se présente sous la forme de deux couches de catalyseurs empilées dans le réacteur, la première couche étant alors capable d'effectuer la déshydratation du glycérol en acroléine et la seconde étant plus adaptée à l'oxydation de l'acroléine ainsi produite en acide acrylique. L'une ou l'autre de ces configurations sera adaptée en fonction de la technologie de réacteur utilisée.

La réaction selon l'invention peut être mise en oeuvre en phase gaz ou en phase liquide, et de préférence en phase gaz. Lorsque la réaction est réalisée en phase gaz, différentes technologies de procédé peuvent être utilisées, à savoir procédé en lit fixe, procédé en lit fluidisé ou procédé en lit fluidisé circulant. Dans les 2 premiers procédés, en lit fixe ou en lit fluidisé, la régénération du catalyseur peut être séparée de la réaction. Elle peut se faire ex-situ, par exemple par extraction du catalyseur et combustion sous air ou avec un mélange gazeux contenant de l'oxygène moléculaire. Dans ce cas, la température et la pression auxquelles est opérée la régénération n'ont pas besoin d'être les mêmes que celles auxquelles est effectuée la réaction. Selon le procédé de l'invention, elle peut se faire en continu in-situ, en même temps que la réaction compte tenu de la présence d'oxygène moléculaire ou d'un gaz contenant de l'oxygène moléculaire dans le réacteur. Dans ce cas, la régénération s'apparente à une inhibition de la désactivation et se fait à la température et à la pression de la réaction.

Dans le procédé à lit fluidisé circulant, le catalyseur circule dans deux capacités, un réacteur et un régénérateur. La vaporisation de la solution de glycérol et la réaction de déshydratation étant endothermiques d'une part, et la réaction d'oxydation ainsi que la régénération du catalyseur consistant en la combustion du coke étant exothermiques d'autre part, les deux systèmes se compensent, ce qui contribue à un bon équilibre thermique du procédé.

La sélection du procédé optimal est faite en fonction de différents critères. Le procédé en lit fixe a l'avantage de la simplicité. Les procédés en lit fluidisé permettent de décharger en continu le catalyseur usagé et de recharger en permanence le catalyseur frais sans arrêter la production avec la possibilité d'être isotherme. Le procédé en lit fluidisé circulant a comme avantage de permettre d'optimiser la sélectivité de la réaction en retournant en permanence au réacteur un catalyseur fraîchement régénéré, tout en compensant l'échange d'énergie entre le réacteur et le régénérateur.

Selon un mode de réalisation particulier de l'invention, le procédé est mis en oeuvre dans un réacteur de type échangeur à plaques. Ce réacteur est constitué de plaques formant entre elles des canaux de circulation pouvant contenir un catalyseur. Cette technologie présente de nombreux avantages en termes d'échange thermique, liés à une capacité d'échange de calories élevée. Ainsi, ce type de réacteur est particulièrement adapté pour évacuer la chaleur facilement dans le cas de réactions exothermiques, ou pour apporter des calories dans des phases de démarrage de réactions ou dans le cas de réactions endothermiques. Plus particulièrement, ce réacteur permet à la fois de chauffer ou refroidir le catalyseur. L'échange thermique est particulièrement efficace avec la circulation d'un fluide caloporteur dans le système. Les plaques peuvent être assemblées par modules qui peuvent être empilés. Ainsi, il est possible d'installer dans une même capacité réactionnelle un premier étage pour la réaction de déshydratation du glycérol en acroléine, suivi d'un second étage pour l'oxydation de l'acroléine. Les modules peuvent être alimentés en flux montant ou en flux descendant, l'empilement des catalyseurs étant choisi de façon à ce que le catalyseur qui se désactive le plus vite soit placé au-dessus de l'autre pour faciliter son remplacement. De plus, les modules peuvent être régulés indépendamment en température pour une meilleure optimisation de la réaction. Ce type de réacteur implique ainsi une grande flexibilité que ce soit au niveau de la taille du réacteur, de sa maintenance, ou du remplacement du catalyseur.

Des systèmes pouvant convenir pour le procédé de l'invention sont par exemple les réacteurs décrits dans les documents EP 995491 ou EP 1147807 dont le contenu est incorporé par référence. Ces réacteurs sont particulièrement adaptés pour la conversion catalytique de milieux réactionnels, spécifiquement des milieux réactionnels gazeux, tels ceux mis en oeuvre dans la présente invention. L'échangeur à plaques utilisé pour la préparation de (méth)acroléine ou d'acide (méth)acrylique par oxydation catalytique de précurseurs en C3 ou C4, décrit dans le document US 2005/0020851, peut convenir également pour la fabrication d'acide acrylique à partir du glycérol, objet de la présente invention.

Ainsi, la présente invention a également pour objet un procédé de fabrication d'acide acrylique en une étape par réaction d'oxydéshydratation du glycérol en présence d'oxygène moléculaire dans un échangeur à plaques.

Les étapes de séparation/purification doivent être adaptées à la production d'acide acrylique, l'acide acrylique ayant un point d'ébullition plus élevé que l'eau et l'acroléine. L'acroléine co-produite peut être soit isolée, soit recyclée pour augmenter les rendements en acide acrylique. Par ailleurs, les sous-produits non sélectifs (autres que l'acroléine et l'acide acrylique) peuvent être récupérés et incinérés, produisant ainsi de la vapeur ou de l'énergie. La valorisation énergétique des sous-produits de la réaction d'oxydéshydratation du glycérol permet de plus de réduire fortement les émissions de gaz à effet de serre du procédé, comparativement au procédé conventionnel d'oxydation sélective du propylène, pour lequel le $CO_2$ produit est issu de carbone fossile lors de l'incinération des sous-produits.

Les conditions expérimentales de la réaction en phase gaz sont de préférence une température comprise entre 250°C et 350°C et une pression comprise entre 1 et 5 bars. Pour éviter des réactions consécutives et la formation de produits indésirables, il est important de limiter le temps de séjour dans le réacteur ; par ailleurs, en augmentant le temps de séjour, il est possible aussi d'avoir des conversions plus fortes. Il est souhaitable notamment d'augmenter le temps de contact (temps de séjour) des réactifs au voisinage du catalyseur pour compenser une diminution du taux de conversion lorsque l'on utilise une température de réaction plus faible. Lorsque l'on utilise un catalyseur faiblement oxydant, une augmentation de la température permet d'améliorer le rendement en acide acrylique.

Le glycérol est disponible sous forme concentrée, mais aussi sous forme de solutions aqueuses plus économiques. Avantageusement, on utilise une solution aqueuse de glycérol de concentration comprise entre 10% et 50% en poids dans le réacteur, de préférence entre 15% et 30%. La concentration ne doit pas être trop élevée afin d'éviter des réactions parasites comme la formation d'éthers de glycérol ou des réactions entre l'acroléine ou l'acide acrylique produits et le glycérol. Par ailleurs, la solution de glycérol ne doit pas être trop diluée en raison du coût énergétique induit par l'évaporation de la solution aqueuse de glycérol. Dans tous les cas, la concentration de la solution de glycérol peut être ajustée en recyclant l'eau produite par la réaction. Afin de réduire les frais de transport et de stockage du glycérol, le réacteur peut être alimenté en solution concentrée de 40 à 100% en poids, la dilution à la teneur optimale étant effectuée par un recyclage d'une partie de la vapeur d'eau produite par la réaction et de l'eau de dilution. De même, la récupération de la chaleur en sortie de réacteur peut aussi permettre de vaporiser la solution de glycérol alimentant le réacteur.

Le glycérol issu de la méthanolyse d'huiles végétales en milieu basique peut contenir certaines impuretés telles que chlorure ou sulfate de sodium, matières organiques non glycérineuses, et du méthanol. La présence de sels de sodium est en particulier préjudiciable pour la réaction catalytique de déshydratation car ces sels sont capables d'empoisonner les sites acides du catalyseur. Un traitement préalable du glycérol par échange ionique peut être envisagé.

Comparativement au procédé conventionnel de préparation d'acide acrylique par oxydation sélective de propylène, l'acide acrylique produit selon le procédé de l'invention peut contenir des impuretés de nature différente ou en quantité différente. Selon l'utilisation envisagée, il pourra être envisagé de purifier l'acide acrylique selon les techniques connues de l'homme du métier.

Les Exemples suivants illustrent la présente invention.


Exemples


Dans les exemples, on utilise un réacteur tubulaire constitué d'un tube de longueur 85 cm et de diamètre intérieur 6 mm pour effectuer la réaction de déshydratation du glycérol en phase gaz à pression atmosphérique. Ce réacteur est placé dans une enceinte chauffée maintenue à la température de réaction choisie. Les catalyseurs utilisés sont broyés et/ou pastillés pour obtenir des particules de 0,5 à 1,0 mm. 10 ml de catalyseur sont chargés dans le réacteur pour former un lit catalytique de longueur 35 cm. Celui-ci est porté à la température de réaction pendant 5 à 10 minutes avant l'introduction des réactifs. Le réacteur est alimenté avec une solution aqueuse à 20% en poids de glycérol avec un débit d'alimentation moyen de 12 ml/h, et avec un débit de 0,8 l/h (13,7 ml/mn) d'oxygène moléculaire pour les exemples selon l'invention, sauf indication contraire. Dans ce cas, la proportion relative O$_2$/glycérol vaporisé/vapeur d'eau est 6/4,5/89,5. La solution aqueuse de glycérol est vaporisée dans l'enceinte chauffée, puis passe sur le catalyseur. Le temps de contact calculé est de l'ordre de 2,9 s. Après réaction, les produits sont condensés dans un piège réfrigéré par de la glace pilée.

Des prélèvements des effluents sont effectués de manière périodique. Pour chaque prélèvement, le débit est interrompu et un léger débit d'azote est envoyé dans le réacteur pour le purger. Le piège en sortie de réacteur est alors remplacé, le débit d'azote est arrêté et le réacteur est remis sous flux de réactif.

Pour chaque expérimentation, la masse totale de produits en entrée et en sortie est mesurée, ce qui permet d'effectuer un bilan massique. De même, les produits formés sont analysés par chromatographie. 2 types d'analyses sont effectués :

- une analyse par chromatographie sur colonne remplie (colonne FFAP 2m*1/8") sur un chromatographe Carlo Erba, muni d'un détecteur TCD. L'analyse quantitative est effectuée avec un étalon externe (2-butanone)
- une analyse par chromatographie sur colonne capillaire (colonne FFAP 50 m*0,25 mm) sur un chromatographe HP6890 muni d'un détecteur FID avec les mêmes échantillons conservés à -15°C.

La première méthode est particulièrement adaptée pour avoir une analyse rapide des produits, et notamment le rendement en acroléine. La seconde méthode est utilisée pour avoir une analyse plus précise de certains sous-produits de la réaction. D'autre part, des analyses par GC-MS ou par chromatographie après silylation ont été effectuées pour confirmer ces résultats.

Les produits ainsi quantifiés sont le glycérol non réagi, l'acide acrylique formé et l'acroléine.

Dans les exemples qui suivent, la conversion du glycérol et les rendements sont définis comme suit :

$$\text{Conversion du glycérol (\%)} = (1 - \text{nombre de moles de glycérol restantes} / \text{nombre de moles de glycérol introduites}) \times 100.$$

$$\text{Rendement en acide acrylique (\%)} = \text{nombre de moles d'acide acrylique produites} / \text{nombre de moles de glycérol introduites.}$$

$$\text{Rendement en acroléine (\%)} = \text{nombre de moles d'acroléine produites} / \text{nombre de moles de glycérol introduites.}$$

Tous les résultats sont exprimés en pourcentages molaires par rapport au glycérol entrant.

Exemple 1

On utilise comme catalyseur (10 ml):

(1) une zircone tungstée (90,7% ZrO$_2$ - 9,3% WO$_3$) de Daiichi Kigenso (référence du fournisseur H1417), soit 17 g
(2) une zircone sulfatée (90% ZrO$_2$-10% SO$_4$) de Daiichi Kigenso (référence du fournisseur H1416), soit 16,5 g
(3) une zircone phosphatée (91,5% ZrO$_2$-8,5% PO$_4$) de Daiichi Kigenso (Réf H1418), soit 12,7 g

La réaction est effectuée en l'absence d'oxygène ou en présence d'oxygène. La température de réaction est 300°C ou 350°C. Les résultats sont indiqués dans le tableau ci-après.

| Catalyseur | (1) | (1) | (1) | (2) | (2) | (3) | (3) | (3) | (3) |
|---|---|---|---|---|---|---|---|---|---|
| Oxygène (ml/mn) | 0 | 13,7 | 13,7 | 0 | 13,7 | 0 | 5,6 | 13,7 | 13,7 |
| Température de réaction (°C) | 300 | 300 | 350 | 300 | 300 | 300 | 300 | 300 | 350 |

(suite)

| Catalyseur | (1) | (1) | (1) | (2) | (2) | (3) | (3) | (3) | (3) |
|---|---|---|---|---|---|---|---|---|---|
| Masse de glycérol introduit en cumulé (g) | 32 | 32 | 42 | 25 | 25 | 25 | 25 | 42 | 50 |
| Conversion du glycérol (%) | 100 | 100 | 100 | 97 | 100 | 100 | 100 | 100 | 100 |
| Rendement en acide acrylique (%) | 0 | 4,5 | 7,6 | - | 0,9 | 0,3* | 0,7 | 1,1 | 0,7 |
| Rendement en acroléine (%) | 72,1 | 53 | 19,2 | 40,6 | 52,5 | 46,7 | 43 | 38 | 14,4 |
| - : indéterminé / * : Attribution incertaine (présence de pics multiples proches) | | | | | | | | | |

Exemple 2

On utilise 11,8 g d'un catalyseur d'oxydation (4) mis en forme par addition de glycérol comme liant.

*Préparation du catalyseur (4)*

La préparation de ce type de catalyseur est décrite dans le brevet US 6,310,240.
Le précurseur est préparé à partir des solutions A et B :
Pour la solution A

| | |
|---|---|
| Eau déminéralisée froide | : 70 kg |
| Paratungstate d'ammonium, APT | : 3,400 kg |
| Métavanadate d'ammonium, MVA | : 2,810 kg |
| Heptamolybdate d'ammonium, HMA | : 11,590 kg |
| Pour la Solution B | |
| Eau déminéralisée froide | : 15 kg |
| Nitrate de Strontium, Sr | : 0,590 kg |
| Nitrate de Cuivre, Cu | : 2,810 kg |

Quand on a obtenu une solution A limpide, on commence la précipitation en versant la solution B dans la solution A à l'aide d'une canne, à un débit de 301/h : (en 30 minutes). La solution B est ajoutée à l'aide d'une pompe doseuse. Le précipité se forme immédiatement et la solution change de couleur.

A la fin de la précipitation (soit 2 heures après le début de la préparation), on évapore l'eau et on obtient un précurseur sec qui est alors précalciné sous air à une température de 225°C.

On prépare une solution d'acide polysilicique :

615 g de solution de silicate de sodium à 28,48 % pds de $Na_2SiO_3$ sont ajoutés à 2885 g d'eau désionisée pour atteindre une solution à 5 % de Si. Cette solution est agitée pendant 10 minutes environ à 1200 tr/min. De la résine échangeuse d'ions HCR-W2H Dowex est ajoutée jusqu'à ce que le pH de la solution soit compris entre 2,5 et 3. Le slurry est filtré sur Büchner muni de papier filtre pendant 10 minutes. Le filtrat (3328 g) récupéré est de couleur brun orangé. 1302 g d'eau déionisée sont rajoutés au filtrat de manière à obtenir une solution contenant 3,6 % d'acide polysilicique. Environ 40 ml d'une solution d'acide sulfurique à 6,9 % sont ajoutés à la solution précédente de manière à descendre le pH en dessous de 2,5. La solution ainsi préparée est stockée à froid.

Le solide précalciné ci-dessus est micronisé de manière à obtenir une poudre de moins de 10 microns et dont le diamètre moyen des particules est de 1 à 1,8 microns.

Un slurry à 34,5 % pds en masse de solide est préparé. Le solide contient 60 % de catalyseur, 30 % de silice colloïdale et 10 % de silice ex-acide polysilicique.

On ajoute à la solution de silice colloïdale de Nalco (50 % pds Nalco 1060) dont le pH de départ est de 8,25, de la résine jusqu'à ce que le pH de la solution soit inférieur à 3. On maintient une agitation de 400 tr/min, le pH final est de 2,0. Le slurry est filtré sur büchner muni de papier filtre. Dans un flacon maintenu dans la glace, on ajoute 200 g de la solution ainsi filtrée, 200 g de solide précalciné et 557,3 g de la solution d'acide polysilicique à 5 % et on mélange le slurry à 800 tr/min pendant 15 min.

L'opération de séchage de ce slurry est effectuée dans un atomiseur Bowen en mode fontaine avec les paramètres suivants :

Température de l'air entrant : 370 °C,
Pression de service de l'air dans la buse : environ 0,5 bars,

Le débit d'alimentation de la suspension (maintenue à 5-10 °C et à un pH inférieur à 4,0): comprise entre 200 et 250 g/min

La pression d'entrée de l'air = 102 mm d'eau.

Pendant l'opération d'atomisation la température de l'air à la sortie descend à 170 °C. Le solide obtenu se présente sous la forme de particules sphériques, de densité comprise entre 1,0 et 1,1 g/ml, de diamètre moyen de l'ordre de 70 microns.

On collecte par tamisage la fraction de granulométrie comprise entre 45 et 150 microns. La calcination est effectuée à 400°C pendant 4 h, en four briqueté et la poudre obtenue est saupoudrée sur un lit de billes de support inerte Norton pour obtenir un rapport de 500 g de poudre pour 2kg de support Norton. A l'issue de la calcination, on récupère la poudre par tamisage. Le catalyseur est mis en forme par addition de glycérol comme liant.

*Résultats*

| | | | | |
|---|---|---|---|---|
| Alimentation sol glycérol à 20% (g/h) | 7,3 | 8,9 | 8,8 | 6,6 |
| Oxygène (ml/mn) | 20 | 20 | 14 | 20 |
| Température de réaction (°C) | 300 | 300 | 300 | 280 |
| Conversion du glycérol (%) | 100 | 100 | 95 | 100 |
| Rendement en acide acrylique (%) | 6,2 | 7,2 | 2,7 | 7,4 |
| Rendement en acroléine (%) | 2 | 4 | 14 | 3 |

Exemple 3

On charge le réacteur avec deux lits catalytiques successifs, le flux gazeux traversant successivement le premier lit puis le second.

Le premier lit catalytique est constitué de 4 ml du catalyseur (1) représentant une masse de 6,2g, le second de 10 ml de catalyseur (4) mis en forme par addition de glycérol comme liant, l'ensemble représentant une masse de 11,8g.

Dans cet exemple, l'oxygène est introduit en amont à raison de 14ml/min ainsi que 8 ml/min d'azote.

*Résultats*

| | | | | |
|---|---|---|---|---|
| Alimentation sol glycérol à 20% (g/h) | 6,2 | 4,2 | 4,8 | 6,4 |
| Oxygène / azote (ml/mn) | 14/8 | 14/8 | 14/8 | 14/8 |
| Température de réaction (°C) | 260 | 260 | 240 | 220 |
| Conversion du glycérol (%) | 100 | 100 | 100 | 100 |
| Rendement en acide acrylique (%) | 34,6 | 31,3 | 43,6 | 1 |
| Rendement en acroléine (%) | < 0,5 | < 0,5 | < 0,5 | > 1 |

Exemple 4

Le premier lit catalytique est constitué de 10 ml du catalyseur (1) représentant une masse de 12,7g, le second de 5 ml de catalyseur (4) mis en forme par addition d'acide acétique comme liant, l'ensemble représentant une masse de 5,25g. Le flux gazeux traverse successivement le premier lit puis le second lit.

*Résultats*

| Alimentation sol glycérol à 20% (g/h) | 9,0 | 8,6 | 9,7 | 9,3 | 9,7 | 9,8 | 9,4 |
|---|---|---|---|---|---|---|---|
| Oxygène (ml/mn) | 14 | 14 | 14 | 14 | 14 | 14 | 14 |
| Température de réaction (°C) | 280 | 280 | 280 | 280 | 280 | 280 | 280 |
| Conversion du glycérol (%) | 100 | 100 | 100 | 100 | 100 | 100 | 100 |
| Rendement en acide acrylique (%) | 41,0 | 74,9 | 63,7 | 66,2 | 69,9 | 71,7 | 73,7 |
| Rendement en acroléine (%) | < 0,5 | < 0,5 | < 0,5 | < 0,5 | < 0,5 | < 0,5 | < 0,5 |

Exemple 5

On reproduit l'exemple 4 avec des débits variables d'oxygène et d'azote.

*Résultats*

| Alimentation sol glycérol à 20% (g/h) | 9 | 9 | 9 | 9 |
|---|---|---|---|---|
| Oxygène / azote (ml/mn) | 8,2 / 6,4 | 8,2 / 6,4 | 5,3 / 9,4 | 6,8 / 6 |
| Température de réaction (°C) | 280 | 260 | 280 | 280 |
| Conversion du glycérol (%) | 100 | 100 | 100 | 100 |
| Rendement en acide acrylique (%) | 71,4 | 37,3 | 57,3 | 70,6 |
| Rendement en acroléine (%) | < 0,5 | 32,5 | 11,7 | < 0,5 |

Exemple 6

On charge le réacteur avec un premier lit catalytique constitué par 10 ml du catalyseur (1) représentant une masse de 17g et un second lit catalytique constitué de 5 ml d'un catalyseur d'oxydation (5), soit une masse de 6g.

*Préparation du catalyseur (5)* : $MoV_{0.33}Nb_{0.11}(oxalates)_{0.30}(NH_4)_{1.15}Si_{0.93}O_x$

Préparation de la solution A (Mo,V)

Dans un bécher, on introduit :

- 265 g d'eau déminéralisée
- 13,3 g de métavanadate d'ammonium ($NH_4VO_3$ - MVA - de GFE lot 9811694)
- 61,0 g d'heptamolybdate d'ammonium ($M_{07}O_{24}(NH_4)_6,4H_2O$ -HMA - de Starck lot 064/001)

Le mélange est chauffé à 80°C, sous agitation jusqu'à obtention d'une solution limpide jaune orangée, soit environ pendant 30 min. Le bécher est recouvert d'un verre de montre pour limiter l'évaporation.
On laisse ensuite, sous agitation, cette solution refroidir jusqu'à la température ambiante.

Préparation de la solution B d'oxalate de niobium

Dans un bécher, on introduit :

- 80 g d'eau déminéralisée
- 12,9 g d'acide oxalique ($C_2H_2O_4$, $2H_2O$ de PROLABO lot G23G)
- 6,4 g d'acide niobique ($Nb_2O_5$, x $H_2O$ de CBMM, 79 % poids après perte au feu)

Le mélange blanchâtre est chauffé à une température située entre 60°C et 70°C sous agitation pendant 2 heures. Un verre de montre est posé sur le bécher pour limiter l'évaporation. On obtient une solution plus claire, que l'on centrifuge 12min à 6200trs/min. Après centrifugation, la solution est à température ambiante et il reste un léger dépôt (inférieur à 5% poids) de solide non dissout, qu'on élimine.

Formation du gel

On ajoute 49,2 g de silice colloïdale (Ludox 40% poids de silice) à la solution A (Mo,V). On ajoute ensuite la solution B d'oxalate de niobium et on obtient immédiatement un gel jaune vif et épais (il faut augmenter le réglage de l'agitateur pour pouvoir l'agiter). A température ambiante, on agite pendant 30 minutes.

Séchage du gel

Le gel obtenu est soutiré et versé dans un plateau recouvert de Téflon, puis mis à l'étuve à 130°C pendant une nuit. On obtient environ 97,6 g de précurseur sec homogène noir brillant.

Pré-calcination

Elle se fait sous flux d'air 50 ml/min.g pendant 4h à 300 °C après une rampe de 2 °C/min. On utilise à chaque fois 30 g de précurseur, qui sont placés dans une capacité en acier d'un volume de 120 ml. La capacité en acier est recouverte d'un couvercle percé d'un trou et est munie d'un puits thermométrique pour suivre la température du solide au cours du traitement thermique. La température est contrôlée avec un thermocouple situé dans le puits thermométrique.

Calcination

Elle se fait sous un débit d'azote d'environ 50 ml/min/g de précurseur, pendant 2h à 600°C après une rampe de 2°C/min. La température est contrôlée avec un thermocouple situé dans le puits thermométrique. L'azote est filtré pour supprimer toute trace d'oxygène et d'humidité, sur des cartouches purificatrices de gaz.

*Résultats*

| Alimentation sol glycérol à 20% (g/h) | 9 | 4,5 | 4,8 | 9 | 9 |
|---|---|---|---|---|---|
| Oxygène / azote (ml/mn) | 6,5 / 7 | 6,5 / 7 | 6,5 / 7 | 6,5 / 7 | 6,5 / 7 |
| Température de réaction (°C) | 280 | 280 | 280 | 325 | 350 |
| Conversion du glycérol (%) | 100 | 100 | 100 | 100 | 100 |
| Rendement en acide acrylique (%) | 25,1 | 29,7 | 35 | 31,4 | 7,3 |
| Rendement en acroléine (%) | 20 | Non dét | 4,4 | 24 | Non dét |

Exemple 7

On utilise deux réacteurs séparés permettant ainsi d'ajuster la température de chaque réaction ainsi que, si nécessaire, la composition des flux gazeux par le biais d'une entrée latérale de gaz entre les deux réacteurs. 10 ml du catalyseur (1) représentant une masse de 17g sont chargés dans le premier réacteur. 7,5 ml d'un catalyseur d'oxydation (6), soit une masse de 9,2g précédés de 2-3 ml de billes de verre sont introduits dans le second réacteur. Le débit d'introduction de la solution aqueuse de glycérol (20 % poids en glycérol) est réglé à 21 g/h.

L'oxygène est introduit en amont des deux réacteurs dont les températures de réaction sont fixées à 280°C.

*Préparation du catalyseur (6)*

Il s'agit d'un catalyseur préparé par enrobage d'un support inerte.

L'enrobeur est mis en rotation grâce à un moteur électrique et est chauffé par une flamme de propane.

On prépare alors les matières premières suivantes :

Support Norton SA5218 en billes de 5 mm 36 kg

Pour la solution A

| | |
|---|---|
| Eau déminéralisée froide | : 70 litres |
| Paratungstate d'ammonium, APT | : 1,700 kg |
| Métavanadate d'ammonium, MVA | : 2,810 kg |
| Heptamolybdate d'ammonium, HMA | : 11,590 kg |
| Pour la Solution B | |
| Eau déminéralisée froide | : 15 litres |
| Nitrate de Strontium, Sr | : 0.590 kg |
| Nitrate de Cuivre, Cu | : 2.810 kg |

La solution A est préparée dans l'enrobeur. On introduit tout d'abord l'eau froide puis on met l'enrobeur en route. La vitesse de l'enrobeur est contrôlée pour éviter tout débordement. On introduit alors successivement le sel de tungstène (APT), le sel de Vanadium (MVA) et le sel de Molybdène (HMA).

On règle alors le débit de gaz (propane) à 2500 l/h et on met l'enrobeur en chauffe.

Pendant ce temps on prépare la solution B. On met les sels en place dans la cuve prévue, on verse l'eau froide, on met en chauffe la cuve et on met l'agitation en route. Le débit de la solution sortant de cette cuve doit être régulier et de 30 l/h (la cuve doit se vider en environ 30 minutes). Une pompe doseuse est utilisée pour s'assurer d'avoir un débit constant pendant la phase de précipitation.

Lorsque la solution A arrive à ébullition (température stable au voisinage de 100 °C), on diminue le débit de propane à 2000 l/h.

On ajoute 10 l d'eau déminéralisée froide dès que la solution est à 100 °C en évitant un débordement de l'enrobeur. Il faut environ une heure pour obtenir une solution limpide de couleur orangée.

Quand on a obtenu une solution A limpide, on commence la précipitation en versant la solution B dans la solution A à l'aide d'une canne, à un débit de 30 l/h : (en 30 minutes). La solution B est ajoutée à l'aide d'une pompe doseuse. On règle alors le débit de gaz (propane) à 1500 l/h. Le précipité se forme immédiatement et la solution change de couleur.

A la fin de la précipitation (soit 2 heures après le début de la préparation), on s'assure qu'il y a assez de place dans l'enrobeur et on ajoute rapidement tout le support. On diminue ensuite le débit de propane à 1000 l/h. L'ajout du support provoque un léger refroidissement de la solution. Quelques minutes plus tard la solution est à nouveau à ébullition. On maintient la solution à ébullition et on laisse la solution s'évaporer. Cette étape prend environ 1 h 30.

A la fin de l'enrobage, on remonte progressivement la température de l'enrobeur, jusqu'à obtenir des billes bien sèches. On ne dépasse pas 108 °C de température des billes. On coupe la chauffe de l'enrobeur avant que cette température ne soit atteinte.

Le taux d'humidité des billes est suivi avec l'aide d'un dessicateur à infrarouges LJ16 de Mettler. On prélève un échantillon d'environ 10 g de billes et on le place dans le dessicateur. Le dessicateur est programmé à 160 °C, réglage de la durée sur 5 minutes, affichage en mode perte de masse en (% poids). On arrête l'enrobage lorsque la perte de masse s'établie entre 2,0 et 2,5 % poids, (soit ½ heure après l'arrêt du chauffage).

On récupère 54 kg de précurseur sec. Des fines sont parfois obtenues au déchargement. Dans le cas présent on récupère 0.734 kg de fines.

La calcination s'effectue en four briquetté dans des barquettes en aluminium munies d'un couvercle. On prépare 6 plateaux de 6 barquettes et on verse 6 fois environ 2 kg de précurseur dans chaque plateau, et on égalise la hauteur. La totalité du précurseur hormis 1.5 kg prélevés pour échantillonnage sont ainsi calcinés.

On place les couvercles sur chaque barquette.

On introduit les plateaux dans le four (qui est déjà à 400 °C), la température maximale atteinte pendant la calcination est de 401°C, la durée de séjour dans le four est de 4 heures.

Au bout de 4 heures, on retire les plateaux du four alors qu'ils sont encore à 400 °C. On laisse alors les plateaux refroidir.

On a ainsi obtenu 48,52 kg après criblage de catalyseur (6) de couleur gris-noir.

Le taux de matière active fixée sur le support, déterminé par décapage chimique est de 27,9 % poids. La densité de remplissage tassée est mesurée sur 500 ml de catalyseur. La valeur obtenue est de 1,37 kg/litre. Le diamètre moyen des grains est de 5,07 mm (moyenne sur 100 grains). Le volume poreux mesuré par porosimétrie au mercure est de 0,1682 ml/g.

*Résultats*

| Alimentation sol glycérol à 20% (g/h) | 21 | 21 | 21 | 21 | 21 | 21 |
|---|---|---|---|---|---|---|
| Oxygène (ml/mn) | 10 | 19 | 32 | 42 | 65 | 88 |
| Température de réaction (°C) | 280 | 280 | 280 | 280 | 280 | 280 |
| Conversion du glycérol (%) | 100 | 100 | 100 | 100 | 100 | 100 |
| Rendement en acide acrylique (%) | 45,2 | 63,7 | 68,4 | 69,7 | 68,4 | 65,7 |
| Rendement en acroléine (%) | 31,1 | 14,2 | 7,7 | 5,4 | 4,0 | 3,9 |

Exemple 8

On reproduit l'exemple 7, mais en faisant varier la température d'oxydation, la température du réacteur de déshydratation étant fixée à 280°C et le débit d'oxygène introduit en amont des deux réacteurs étant constant.

*Résultats*

| Alimentation sol glycérol à 20% (g/h) | 21 | 21 | 21 | 21 |
|---|---|---|---|---|
| Oxygène (ml/mn) | 42 | 42 | 42 | 42 |
| Température du réacteur d'oxydation (°C) | 260 | 280 | 300 | 320 |
| Conversion du glycérol (%) | 100 | 100 | 100 | 100 |
| Rendement en acide acrylique (%) | 63,2 | 69,7 | 65,7 | 65,3 |
| Rendement en acroléine (%) | 9,5 | 5,4 | 3,9 | 1,7 |

Exemple 9

On reproduit l'exemple 7, mais en faisant varier le débit d'oxygène au niveau des deux réacteurs. Une partie de l'oxygène nécessaire est introduite en amont des deux réacteurs (Oxygène 1), le complément est apporté par l'entrée latérale placée entre les deux réacteurs (Oxygène 2). La température est fixée à 280°C.

*Résultats*

| Oxygène 1 (ml/mn) | 42 | 9 | 20,2 | 20,2 | 88 | 42 |
|---|---|---|---|---|---|---|
| Oxygène 2 (ml/mn) | 0 | 32 | 21 | 21 | 0 | 45 |
| Température de réaction (°C) | 280 | 280 | 280 | 280 | 280 | 280 |
| Conversion du glycérol (%) | 100 | 100 | 100 | 100 | 100 | 100 |

(suite)

| Rendement en acide acrylique (%) | 69,7 | 62,2 | 69,3 | 67,3 | 65,7 | 68,4 |
|---|---|---|---|---|---|---|
| Rendement en acroléine (%) | 5,4 | 2,8 | 2,8 | 3,3 | 3,9 | 1,9 |

Exemple 10

On reproduit l'exemple 7, mais l'oxygène est substitué par l'air. L'oxygène nécessaire, pur ou en mélange, est introduit soit complètement en amont des deux réacteurs (gaz 1), soit un complément est apporté par l'entrée latérale placée entre les deux réacteurs (gaz 2).

*Résultats*

| Gaz 1 (ml/mn) | 42-$O_2$ | 88-$O_2$ | 40,8-air | 217-air |
|---|---|---|---|---|
| Gaz 2 (ml/mn) | 0 | 0 | 32-$O_2$ | 0 |
| Température, °C | 280 | 280 | 280 | 280 |
| Conversion du glycérol (%) | 100 | 100 | 100 | 100 |
| Rendement en acide acrylique (%) | 69,7 | 65,7 | 66,1 | 64,1 |
| Rendement en acroléine (%) | 5,4 | 3,9 | 2,9 | 1,7 |

Exemple de référence

Les exemples 1 à 5 de la demande WO 05/073160 sont reproduits et sont appelés exemples 1 à 5 de référence Les catalyseurs sont préparés selon le mode opératoire décrit, à l'exception de la taille du matériau support qui est plus faible (0,5-1,6 mm), la réaction étant réalisée dans des réacteurs de diamètre plus petit (6 mm au lieu de 25 mm). Des fours sont utilisés comme systèmes chauffants.

| | Exemple 1 de référence | Exemple 2 de référence | Exemple 3 de référence | Exemple 4 de référence | Exemple 5 de référence |
|---|---|---|---|---|---|
| Nombre de réacteurs | 2 | 2 | 2 | 1 | 1 |
| Température, °C | 1ère étape : 295 2ème étape :270 | 1ère étape : 290 2ème étape :270 | 1ère étape: 292 2ème étape:270 | 295 | 294 |
| % glycérol dans la sol aqueuse | 85 | 91 | 92 | 98 | 91 |
| Alimentation, % Glyc./eau/$O_2$/$N_2$ | 10/9/6/75 | 14/7/0/79 | 10/4/6/80 | 10/1/6/83 | 10/5/6/79 |
| Durée en heures | 5,5 | 5,5 | 5,7 | 5,5 | 5 |
| Conversion glycérol, % | 100 | 99,5 | 99 | 99,6 | 99,5 |
| Rendement acide acrylique, % | 3,89 | 0,39 | 0 | 3,74 | 4,10 |
| Rendement en acroléine, % | 23,63 | 9,51 | 35,47 | 12,84 | 20,76 |

Les résultats obtenus mettent en évidence la formation d'acroléine en quantité plus importante que l'acide acrylique, ce qui indique une mauvaise activité du catalyseur pour la réaction d'oxydation. Par ailleurs, les rendements obtenus en acide acrylique et acroléine sont nettement inférieurs à ceux indiqués dans la demande WO 05/073160.

**Revendications**

1. Procédé de fabrication d'acide acrylique en une étape par réaction d'oxydéshydratation du glycérol en présence d'oxygène moléculaire, le glycérol étant sous forme de solution aqueuse de concentration comprise entre 10% et 50% en poids dans le réacteur.

2. Procédé selon la revendication 1 **caractérisé en ce que** la concentration de la solution aqueuse de glycérol est comprise entre 1.5% et 30% dans le réacteur.

3. Procédé selon la revendication 1 ou 2 **caractérisé en ce que** la réaction est réalisée en phase gaz dans un réacteur à lit fixe, à lit fluidisé ou à lit fluidisé circulant.

4. Procédé selon la revendication 3 **caractérisée en ce que** la réaction est effectuée à une température comprise entre 250°C et 350°C.

5. Procédé de fabrication d'acide acrylique en une étape par réaction d'oxydéshydratation du glycérol en présente d'oxygène moléculaire dans un échangeur à plaques.

6. Procédé selon la revendication 5, caractérisé en ce le glycérol est sous forme de solution aqueuse de concentration comprise entre 10% et 50% en poids dans le réacteur, de préférence entre 15% et 30% en poids.

7. Procédé selon l'une des revendications 1 à 6, **caractérisé en ce que** l'oxygène moléculaire est sous forme d'air ou sous forme d'un mélange de gaz contenant de l'oxygène moléculaire.

8. Procédé selon l'une des revendications 1 à 7, **caractérisé en ce que** le glycérol est dilué dans le réacteur par un recyclage d'une partie de la vapeur d'eau produite par la réaction et de l'eau de dilution, le réacteur étant alimenté avec des solutions concentrées de 40 à 100 % en poids de glycérol.

9. Procédé selon l'une des revendication 1 à 8, **caractérisé en ce que** la réaction est effectuée en présence d'un catalyseur unique capable de catalyser à la fois la réaction de déshydratation et la réaction d'oxydation, ou en présence d'un mélange de catalyseurs, chacun étant capable d'effectuer l'une de ces deux réactions.

10. Procède selon la revendication 9 **caractérisé en ce que** le catalyseur unique ou le catalyseur d'oxydation est un solide contenant au moins un élément choisi dans la liste Mo, V, W, Re, Cr, Mn, Fe, Co, Ni, Cu, Zn, Sn, Te, Sb, Bi, Pt, Pd, Ru, Rh, présent sous la forme métallique ou sous forme d'oxyde, de sulfate ou de phosphate.

**Claims**

1. A process for manufacturing acrylic acid in a single step by oxydehydration reaction of glycerol in the presence of molecular oxygen, the glycerol being in the form of an aqueous solution with a concentration of between 10% and 50% by weight in the reactor.

2. The process as claimed in claims 1, **characterized in that** the concentration of the aqueous solutions of glycerol is between 15% and 30% by weight in the reactor.

3. The process as claimed in claim 1 or 2, **characterized in that** the réaction is performed in the gas phase in a fixed-bed reactor, a fluidized-bed reactor or a circulating fluidized-bed reactor.

4. The profess as claimed in claim 3, **characterized in that** the reaction is performed at a temperature of between 250°C and 350°C.

5. A profess for manufacturing acrylic acid in a single step by oxydehydration reaction of glycerol in the presence of molecular oxygen in a plate exchanger.

**6.** The process as claimed in claim 5, **characterized in that** the glycerol is in the form of an aqueous solution with a concentration of between 10% and 50%, preferably between 15% and 30% by weight in the reactor.

**7.** The process as claimed in one of claims 1 to 6, **characterized in that** the molecular oxygen is in the form of air or in the form of a mixture of gases containing molecular oxygen.

**8.** The process as claimed in one of claims 1 to 7, **characterized in that** the dilution of the glycerol solution in the reactor is performed by recycling part of the steam produced by the reaction and of the dilution water, the reactor being fed with concentrated solutions of from 40% to 100% by weight of glycerol.

**9.** The process as claimed in one of claims 1 to 8, **characterized in that** the reaction is performed in the presence of a single catalyst capable of catalyzing both the dehydration reaction and the oxidation reaction, or in the presence of a mixture of catalysts, each being capable of effecting one of these two reactions.

**10.** The process as claimed in claim 9, **characterized in that** the single catalyst or the oxidation catalyst is a solide containing at least one element chosen from the list Mo, V, W, Re, Cr, Mn, Fe, Co, Ni, Cu, Zn, Sn, Te, Sb, Bi, Pt, Pd, Ru and Rh, present in metallic form or in the form of oxide, sulfate or phosphate.

**Patentansprüche**

**1.** Einstufiges Verfahren zur Herstellung von Acrylsäure durch Oxydehydratisierung von Glycerin in Gegenwart von molekularem Sauerstoff, wobei das Glycerin in Form einer wässrigen Lösung mit einer Konzentration zwischen 10 und 50 Gew.-% in dem Reaktor vorliegt.

**2.** Verfahren nach Anspruch 1, **dadurch gekennzeichnet, dass** die Konzentration der wässrigen Glycerinlösung in dem Reaktor zwischen 15 und 30 % liegt.

**3.** Verfahren nach Anspruch 1 oder 2, **dadurch gekennzeichnet, dass** die Reaktion in der Gasphase in einem Festbettreaktor, Wirbelschichtreaktor oder einem Reaktor mit zirkulierender Wirbelschicht durchgeführt wird.

**4.** Verfahren nach Anspruch 3, **dadurch gekennzeichnet, dass** die Reaktion bei einer Temperatur zwischen 250 und 350 °C durchgeführt wird.

**5.** Einstufiges Verfahren zur Herstellung von Acrylsäure durch Oxydehydratisierung von Glycerin in Gegenwart von molekularem Sauerstoff in einem Plattenwärmetauscher.

**6.** Verfahren nach Anspruch 5, **dadurch gekennzeichnet, dass** das Glycerin in Form einer wässrigen Lösung mit einer Konzentration zwischen 10 und 50 Gew.-% und vorzugsweise 15 bis 30 Gew.-% in dem Reaktor vorliegt.

**7.** Verfahren nach einem der Ansprüche 1 bis 6, **dadurch gekennzeichnet, dass** das molekulare Sauerstoff in Form von Luft oder in Form eines Gasgemisches, das molekularen Sauerstoff enthält, vorlegt.

**8.** Verfahren nach einem der Ansprüche 1 bis 7, **dadurch gekennzeichnet, dass** das Glycerin in dem Reaktor durch Rückführung eines Teils des durch die Reaktion gebildeten Wassers und des Verdünnungswassers verdünnt wird, wobei dem Reaktor konzentrierte Lösungen von 40 bis 100 Ges.-% Glycerin zugeführt werden.

**9.** Verfahren nach einem der Ansprüche 1 bis 8, **dadurch gekennzeichnet, dass** die Umsetzung in Gegenwart eines einzigen Katalysators durchgeführt wird, der befähigt ist, sowohl die Dehydratisierung als auch die Oxidation zu katalysieren, oder in Gegenwart eines Gemisches von Katalysatoren, die jeweils befähigt sind, eine der beiden Reaktionen durchzuführen.

**10.** Verfahren nach Anspruch 9, **dadurch gekennzeichnet, dass** der einzige Katalysator oder der Oxidationskatalysator ein Feststoff ist, der zumindest ein Element enthält, das unter Mo, V, W, Re, Cr, Mn, Fe, Co, Ni, Cu, Zn, Sn, Te, Sb, Bi, Pt, Pd, Ru, Rh ausgewählt ist, die in metallischer Form oder in Form von Oxid, Sulfat oder Phosphat vorliegen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 695931
- US 2558520 A
- WO 9905085 A
- US 5387720 A
- WO 2005073160 A
- EP 995491 A
- EP 1147807 A
- US 20050020851 A
- US 6310240 B
- WO 05073160 A